# EUROPEAN PATENT SPECIFICATION

(11) **EP 0 428 791 B1**
(45) Date of publication and mention of the grant of the patent: **19.10.1994**
(21) Application number: 89202983.6
(22) Date of filing: 24.11.1989
(51) Int. Cl.: C07C 25/24, C07C 17/34, B01J 21/04

(54) **Process for using an improved catalyst for dehydrohalogenation**
Katalytisches Dehydrohalogenierungsverfahren
Procédé catalytique de déshydrohalogénation

(43) Date of publication of application: 29.05.1991
(73) Proprietor: DowElanco, Indianapolis, Indiana 46268-1189 (US)
(72) Inventor: Klipa, Dennis K., Midland Michigan 48640 (US); Dewald, James R., Bay City Michigan 48706 (US)
(74) Representative: Smulders, Theodorus A.H.J., Ir.

(56) References cited:
- EP-A- 0 027 155
- WO-A-88/09319
- US-A- 3 607 955
- US-A- 4 188 346
- US-A- 4 205 015

## Description

This invention relates to processes for preparing α(alpha)-substituted styrenes. Alpha-(1-haloalkyl)styrenes are taught in U.S. Patent 3,391,203 to be useful as parasiticides and insecticides. They are also known to be useful intermediates in the manufacture of other biologically-active compounds such as herbicides, as taught in U.S. Patent No. 3,373,011. In preparing α-substituted styrenes, U.S. Patent 4,188,346 discloses that the Lewis acid SbCl₅ alone was a good catalyst, but it was later shown to be difficult to remove and detrimental to subsequent epoxidations. U.S. Patent 4,205,015 discloses a method for preparing α-substituted styrenes using a Lewis acid catalyst such as AlCl₃ deposited on an inert support such as silica gel or alumina. However, this catalyst suffers the shortcomings of requiring additional time and expense to prepare and has safety problems associated with the release of undesirably high levels of hydrogen halide.

In view of the deficiencies of prior art methods, it would be highly desirable to provide an improved method for making α-substituted styrenes.

In accordance with the present process invention, α-substituted styrenes are advantageously obtained by reacting, in the absence of an added Lewis acid, an α-haloalkylbenzene with dried γ(gamma)-alumina, the γ-alumina serving to catalyze dehydrohalogenation of the α-haloalkylbenzene to produce an α-substituted styrene. The present process has the advantage of utilizing a catalyst which requires less time and expense to prepare compared with other previously taught processes. Another advantage of the present process is the improvement in safety over other known processes by eliminating the levels of noxious fumes, such as hydrogen halide, produced during catalyst preparation. Another advantage of the present process is that it simplifies the preparation of the catalyst over other previously taught processes. The α-substituted styrenes produced in the practice of this process are useful as biologically active compounds as described hereinbefore and as intermediates in the preparation of other biologically-active compounds. Still yet another advantage of the present invention is that it provides a method for making such α-substituted styrenes in a yield and purity as good as or better than that taught by other processes while also enjoying the above listed advantages.

α-haloalkybenzenes reacted in accordance with the present invention are represented by formula (I) 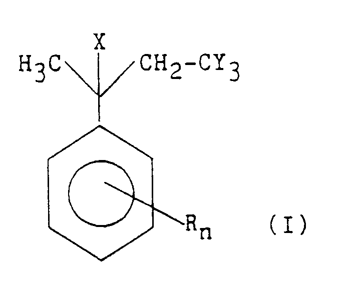
wherein X, R and Y are independently halogens, and n is from 1 to 3. Most preferably, n is 2 and each R is individually chloro substituted at the 3 and 5 positions on the ring. Preferably, X is chloro or bromo, most preferably chloro. Each Y is independently the same or different halogen, so that Y₃ maybe such as -Cl₃, -Br₃, - F₃, -I₃, -Cl₂Br, -Br₂Cl or -Cl₂I. More preferably, Y₃ is -Cl₃ or -Br₃, and most preferably is -Cl₃.

Examples of especially preferred α-haloalkylbenzenes include 3-(3,5-dichlorophenyl)-1,1,1,3-tetrachlorobutane (DCTCB) and 3-bromo-3-(3,5-dichlorophenyl)-1,1,1-trichlorobutane.

α-Haloalkylbenzenes are suitably prepared by any known process. For example, α-methylstyrene or a substituted α-methylstyrene, prepared in accordance with one of the procedures described in U.S. Patent 2,816,934, is reacted with a polyhaloalkane such as carbon tetrachloride, bromotrichloromethane, methylene chloride or dichloronitromethane, in the presence of an amine and cuprous chloride, by the procedure described in U.S. Patent 3,454,657, to produce a desired α-haloalkylbenzene.

Suitable γ-alumina catalysts include χ(chi)-alumina, boehmite or any combination thereof. The α(alpha), κ(kappa), δ(delta), or ϑ(theta) aluminas will not promote dehydrohalogenation as well as γ-alumina and thus are not as practical for commercial applications.

The γ-alumina must be dried to the proper moisture content to provide the proper catalytic action for dehydrohalogenation. Moisture content of the γ-alumina is determined, for example, by comparing the difference in weight between untreated γ-alumina and γ-alumina heated in a vacuum oven at a temperature of 150°C at a pressure of 30 torr (4 kilopascals) for 24 hours. γ-Alumina has a moisture content from zero to 12 percent by weight. More preferably the γ-alumina has a moisture content of 2 to 6 percent by weight, most preferably a moisture content of about 3 percent by weight. Advantageously, the γ-alumina is in the form of a particulate solid, preferably-one having an average particle diameter in the range of 80 to 200 mesh [0.074 to 0.177 millimeters (mm)].

The γ-alumina may be made by heating gibbsite at temperatures of from 100 to 150°C. Gibbsite is a naturally occurring polymorphic form of aluminum hydroxide, Al(OH)₃, or aluminum trihydrate, Al₂O₃·3H₂O, as defined in *X-Ray Identificationand Crystal Structures of Clay Minerals*, G.W. Brindley, (ed.), chapter 10, pages 244-256. Alternatively, γ-alumina may be obtained commercially as F-1™ alumina (Aluminum Company of America). X-ray diffraction analysis of the F-1™ alumina showed it to be a γ-type alumina having primarily two phases: χ-alumina and boehmite alumina.

Drying the γ-alumina to the proper moisture content is critical to the present invention since the presence of water in or on the γ-alumina will inhibit the dehydrohalogenation process. The γ-alumina may be dried by distilling off the water with an organic solvent to form an azeotrope mixture of the water/organic solvent. Halocarbon or hydrocarbon solvents may be used to form the azeotrope mixture. In practicing the invention, the amount of water remaining in the azeotrope mixture was determined by visually observing the separated phases of the solvent and water condensed on the glass condenser.

Hydrocarbon solvents for drying the γ-alumina preferably include benzene, toluene, ethylbenzene, heptane, decane, cyclohexane, or mixtures thereof. Halocarbon solvents preferably include chloroform, carbon tetrachloride, chlorobenzene, dichlorobenzene, trichlorobenzene, 1,1,1-trichloroethane, 1,2-dichloroethane, or mixtures thereof. Most preferably, the halocarbon solvent is chlorobenzene or carbon tetrachloride.

Alternatively, the γ-alumina may be dried by heating it in a vacuum oven at a temperature of 150°C at a pressure of about 30 torr (4 kilopascals) for 24 hours.

The process for preparing any of the α-substituted styrenes from the requisite haloalkyl benzene compounds may be conducted neat, i.e., in the absence of a solvent. Preferably, however, the process is performed using a solvent, such as any of the hydrocarbon or halocarbon solvents listed above for drying the γ-alumina.

In the practice of the present invention, a desired α-substituted styrene is prepared by reacting the requisite α-haloalkylbenzene with γ-alumina at an elevated temperature, the γ-alumina serving to catalyze the dehydrohalogenation of the α-haloalkylbenzene to produce the corresponding α-substituted styrene. The γ-alumina may constitute between 0.1 to 99 percent by weight of the combined weight of the γ-alumina and α-haloalkylbenzene present in the reaction mixture. More preferably the γ-alumina is between 1 to 15 percent of the weight of γ-alumina and the α-haloalkylbenzene in the reaction mixture. Most preferably the weight of the γ-alumina is between 5 to 10 percent by weight of the γ-alumina and α-haloalkylbenzene.

Preferably, the catalyzed reaction is completed in a time of 0.5 to 16 hours to give the desired α-substituted styrene. More preferably, the reaction is completed in 5 to 12 hours.

In carrying out the catalyzed reaction the γ-alumina may be utilized in the form of a fixed bed through which the α-haloalkylbenzene is passed or circulated. The fixed bed is prepared by mounting a fritted support permeable to fluids within a tube. The γ-alumina is placed atop the fritted support and another fritted support is placed atop the alumina. The solvent containing the α-haloalkylbenzene or the α-haloalkylbenzene alone, if being reacted neat, is then passed upwardly through the tube to contact the γ-alumina, forming the desired α-substituted styrene. Alternatively, and more preferably, the γ-alumina is used in the form of a slurry or a mixture thereof with solvent medium containing the α-haloalkylbenzene.

The process for preparing the α-substituted styrene may be advantageously conducted at a temperature in the range of 40 to 160°C. More preferably the process is conducted at temperatures between 75 to 90°C, most preferably at about 82°C.

The process advantageously may be performed at ambient pressures of about 760 torr (101 kilopascals). Preferably the process is performed at lower pressures of 40 to 760 torr (40 to 101 kilopascals). At lower pressures the hydrogen halide gas, a by-product of the reaction, is removed from the γ-alumina, thus enhancing the rate of formation of the α-substituted styrene.

After the α-haloalkylbenzene is contacted with the γ-alumina, the reaction begins immediately, as evidenced by evolution of hydrogen halide gas. Hydrogen halide gas can be detected by contacting the evolving gas with NH₃ vapors; in the case of HCl, a white smoke of NH₄Cl is easily observed. Alternatively, wet litmus paper can be used to detect evolution of hydrogen halide. Where the γ-alumina is used as a slurry, the reaction mixture is agitated sufficiently to keep the γ-alumina in suspension until the dehydrohalogenation reaction is completed.

After formation of an α-substituted styrene by the present invention, the product is separated from the γ-alumina catalyst by filtration and then, if necessary, by fractionally distilling off any solvents that may be present.

The product of the dehydrohalogenation reaction is primarily an α-substituted styrene wherein the benzylic halogen and the hydrogen on an adjacent carbon have been eliminated as HCl. In embodiments of particular interest, the α-substituted styrene is represented by formula (II): 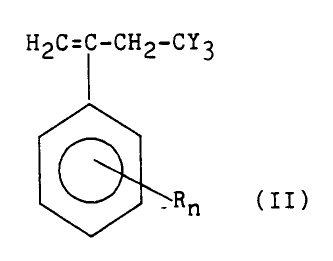
wherein: R, Y and n are defined as hereinbefore. An especially preferred α-substituted styrene is 2-(3,5-dichlorophenyl)-4,4,4-trichloro-1-butene (DCTCS).

The following examples are given to illustrate the invention and should not be construed as limiting its scope. All percentages in the examples are by weight unless otherwise indicated.

In the Examples the following terms are used:
DCTCB = 3-(3,5-dichlorophenyl)-1,1,1,3-tetrachlorobutane
DCTCS = 2-(3,5-dichlorophenyl)-4,4,4-trichloro-1-butene
GLC = gas liquid chromatography

### Example 1.Preparation of DCTCS

### A. Preparation of DCTCB

A 3-necked, round bottomed flask (1 liter) fitted with reflux condenser, internal thermometer, addition funnel, mechanical stirrer and heating mantle was charged with 515 milliliters (ml) CCl₄ and 0.53 grams (g) CuCl and the mixture was heated to reflux (78°C). A solution of 200 g of 3,5-dichloro-α-methylstyrene and 5.61 ml *t*-butylamine was added. The mixture was refluxed for 8.5 hours. After HCl sparge and suction filtration, the filtrate volume was 660 ml. The filtrate was evaporated at 50-60°C with an aspirator vacuum until the solvent was removed. Analysis of this residue by GLC showed that it contained 94.5 percent DCTCB, represented by the formula:

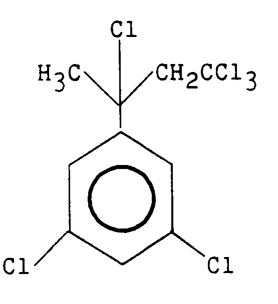

### B. Preparation of DCTCS

To a 3-necked, round bottomed flask fitted with a thermometer, temperature controller, heating mantle, reflux condenser, addition funnel and mechanical stirrer was added CCl₄ and F-1 alumina, which was previously dried at a temperature of 150°C under reduced pressure. The mixture was heated to reflux and DCTCB as prepared above was added. After a short period of time vapors of HCl were observed exiting the reflux condenser. When the reaction was complete, as judged by GLC analysis, the alumina was removed by filtration and the CCl₄ was evaporated under reduced pressure to give the product DCTCS, i.e., 2-(3,5-dichlorophenyl)-4,4,4-trichloro-1-butene represented by the formula:

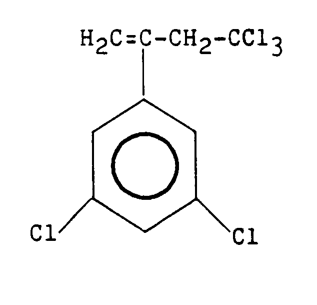

### Example 2. Preparation of DCTCS

To a 3-necked, round bottomed flask fitted with thermometer, temperature controller, heating mantle, reflux condenser, addition funnel and mechanical stirrer was added CCl₄ and F-1™ alumina. The F-1™ alumina catalyst was dried by distilling off the H₂O/CCl₄ azeotrope mixture until only CCl₄ vapors were coming overhead. To this *in situ* prepared catalyst in CCl₄, which is maintained at the appropriate reaction temperature, was added DCTCB prepared in accordance with Example 1. After a short period of time, vapors of HCl were observed exiting the flux condenser. When the reaction was complete, as judged by GLC analysis, the alumina was removed by filtration and the CCl₄ was evaporated under reduced pressure to give crude DCTCS.

### Example 3. Preparation of DCTCS

To a two liter, 3-necked, round bottomed flask fitted with a reflux condenser, mechanical stirrer, thermometer, temperature controller, heating mantle and addition funnel was added F-1™ alumina (53.3 g) and CCl₄ (467 ml). The mixture was brought to reflux and H₂O was seen condensing on the coils of the condenser. The addition of a solution of DCTCB (400 g) in CCl₄ (267 g) was started. After 40 ml of DCTCB was added, no HCl vapor was observed. The H₂O present was then removed by azeotropic distillation with CCl₄. About 65 ml of CCl₄ was distilled off and then 65 ml of fresh dry CCl₄ was recharged to the round bottomed-flask. When the 65 ml of wet CCl₄ had been removed the reaction started. After 80 minutes a sample of the reaction mixture was taken which showed very little DCTCB. The heating mantle was removed after a total reaction time of 100 minutes. After cooling, the reaction mixture was filtered to remove the alumina. The solvent was evaporated under reduced pressure. The resulting DCTCS product had an assay of 85 percent.

### Example 4. Preparation of DCTCS

To a one liter, 3-necked round bottomed flask fitted with an addition funnel, reflux condenser, thermometer, temperature controller and heating mantle was added F-1™ alumina (36 g) and CCl₄ (315 ml). The catalyst slurry was stirred and heated to reflux. A total of 60 ml of CCl₄ was distilled off to remove H₂O and then 60 ml of fresh dry CCl₄ was added. The addition of a 71 percent solution of crude DCTCB in CCl₄ (270 g) was started but no HCl came off immediately. Additional water was observed collecting on the coils of the reflux condenser, so 5 ml more CCl₄ was distilled off and the condenser was removed, rinsed with dry acetone, and replaced. HCl evolution then started and the DCTCB was then completely added in about 5 minutes. After 90 minutes of reaction time, the heating mantle was removed and analysis of the reaction mixture showed no DCTCB remaining. The reaction mixture was cooled to below 30°C and filtered to remove the alumina. The filter cake was washed once with 100 ml CCl₄ and the filtrates were combined. The solvent was removed under reduced pressure to give a product with DCTCS assay of 88 percent.

### Example 5. Preparation of DCTCS

The procedure of Example 4 was repeated except that recrystallized DCTCB was used. After replacing the 60 ml of CCl₄ distilled off at the outset, the DCTCB addition was started but no HCl was observed again. When 5 ml more CCl₄ was distilled off and the condenser was rinsed with acetone and dried before being replaced, HCl evolution began. The addition of the DCTCB solution (270 g DCTCB in 180 g CCl₄) was completed in about 5 minutes. After 1 hour the HCl evolution was slow and a sample taken for analysis showed no DCTCB remaining. The heat was removed after 1 hour and 20 minutes reaction time. The reaction mixture was cooled to below 30°C and filtered to remove the alumina catalyst. The cake was washed once with 100 ml CCl₄ and the filtrates were combined. The solvent was removed under reduced pressure to give crude DCTCS with a 93.7 percent assay.

### Example 6. Preparation of DCTCS

To a 250 ml 3-necked flask fitted with a thermometer, temperature controller, heating mantle, mechanical stirrer, reflux condenser and addition funnel was added 70 ml of CCl₄ and dried F-1™ alumina (8.0 g). The mixture was brought to reflux and after about 30 percent of a solution of 60 g DCTCB in 84.5 g CCl₄ had been added, the addition was stopped to allow reflux. Within 3 minutes of the start of the addition of DCTCB solution, HCl was observed exiting the top of the condenser. Addition of reactant was completed in about 5 minutes. After 45 minutes of reaction, analysis of a sample of the reaction mixture showed no DCTCB remaining. After another 15 minutes, heating was stopped and the mixture was cooled and then filtered to remove the alumina. The solvent was removed under reduced pressure to give a product, which had a DCTCS assay of 88.7 percent.

### Example 7. Preparation of DCTCS

The procedure of Example 6 was repeated, but only 3.6 g of dried F-1™ alumina was used. Within 3 minutes of initial addition of DCTCB to the F-1™ alumina the HCl was coming out of the condenser. After 45 minutes the HCl evolution was evident (visually by fuming white smoke NH₃ vapors) and was faster than that of Example 6 which was run at the same time. The heat was removed from the reaction mixture and the stirring stopped after 1 hour. Analysis of the reaction sample showed 1 percent DCTCB remaining. The reaction mixture was filtered to remove the alumina. The solvent was removed under reduced pressure to give a product with DCTCS assay of 88.4 percent.

### Example 8. Preparation of DCTCS

The catalyst used was F-1™ alumina dried in a vacuum oven at 150°C/20 torr (2.67 kilopascals) for 24 hours. To a 250 ml round bottomed 3-necked flask fitted with a mechanical stirrer, thermometer, temperature controller, heating mantle, addition funnel and reflux condenser was added CCl₄ (70 ml) and F-1™ alumina (3.6 g). The mixture was brought to 70°C ± 3°C and a 71 percent solution of DCTCB in CCl₄ (84.5 g) was added over a 5 minute period. After 10 minutes the first traces of HCl were observed. Analysis of samples taken showed 57 percent conversion after 2 hours and 84 percent conversion after 4 hours to DCTCS.

### Example 9. Preparation of DCTCS

The procedure of Example 8 was repeated except only 1.8 g of F-1™ alumina was added. Addition of 71 percent solution of DCTCB in CCl₄ (84.5 g) was carried out over a 5 minute period. After 15 minutes the first very faint whisps of HCl could be detected. Analysis of samples showed 8 percent conversion to DCTCS after 2 hours and 16 percent conversion after 4 hours. The solution of DCTCB was from the same source as that used in Example 8.

### Example 10. Preparation of DCTCS

To a 250 ml 3-necked, round bottomed flask fitted with a reflux condenser, temperature controller, thermometer, heating mantle and mechanical stirrer was added CCl₄ (70 ml) and a 71 percent solution of DCTCB in CCl₄ (84.5 g). This was heated to 70°C and the F-1™ alumina catalyst (3.6 g) was added all at once. Analysis of samples taken at 35, 95 and 120 minutes showed conversions to DCTCS of 53, 79 and 83 percent, respectively.

### Example 11. Preparation of DCTCS

The procedure of Example 10 was repeated except that the reaction was held at 70°C and the solvent refluxed by reducing the pressure. The catalyst was again solvent-added all at once. Analysis of samples taken at 35, 95 and 120 minutes showed conversions to DCTCS of 67, 94 and 98 percent, respectively.

Upon repeating Examples 1-11 using γ-alumina prepared from gibbsite according to the techniques described hereinbefore, substantially the same excellent results are obtained with respect to yield and purity of α-substituted styrene produced.

## Claims

1. A process for preparing an α-substituted styrene of the formula 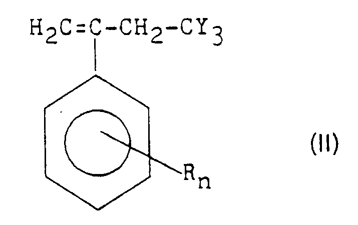 wherein: R represents halogen; n is from 1 to 3; and each Y independently represents halogen;
which comprises reacting, in the absence of an added Lewis acid, γ-alumina catalyst with a moisture content between 0 to 12 percent by weight with an α-haloalkylbenzene of the formula 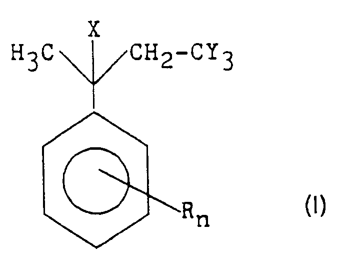 wherein: X is halogen; and R, n and Y are defined as before.

2. The process of Claim 1 in which R is chloro. X is chloro or bromo, and Y₃ is -Cl₃ or -Br₃.

3. The process of Claim 2 in which n is 2 and each R is individually chloro substituted at the 3- and 5-positions on the phenyl ring.

4. The process of any one of the preceding claims in which the γ-alumina is χ-alumina, boehmite or a combination thereof.

5. The process of any one of the preceding claims in which the α-haloalkylbenzene is contacted with the γ-alumina in the presence of a hydrocarbon or halocarbon solvent.

6. The process of any one of the preceding claims in which the γ-alumina is obtained by heating gibbsite at a temperature of 100 to 150°C.

7. The process of Claim 5 in which the γ-alumina is obtained by azeotropically removing water by distilling the hydrocarbon or halocarbon solvent.

8. The process of any one of the preceding claims which is performed at temperatures between 40 and 160°C.

## Patentansprüche

1. Verfahren zur Herstellung eines α-substituierten Styrols der Formel 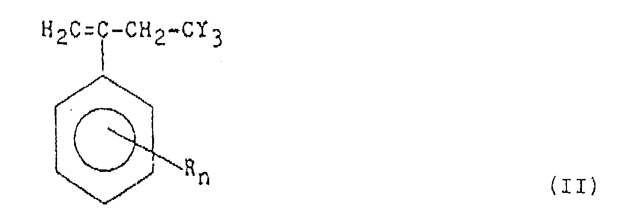 wobei R ein Halogen darstellt; n eine Zahl von 1 bis 3 ist und jedes Y unabhängig voneinander, Halogen darstellt;
welches - in Abwesenheit einer zugegebenen Lewis-Säure - γ-Aluminiumoxid-Katalysator mit einem Feuchtigkeitsgehalt zwischen 0 und 12 Gew.-% mit einem α-Halogenalkylbenzol der Formel 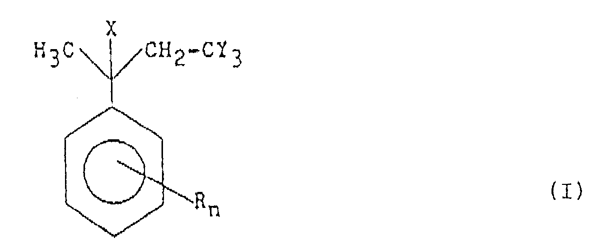 wobei X ein Halogen darstellt und R, n und Y wie oben definiert sind, zur Reaktion bringen umfaßt.

2. Verfahren nach Anspruch 1, wobei R Chlor, X Chlor oder Brom und Y₃ -Cl₃ oder -Br₃ ist.

3. Verfahren nach Anspruch 2, wobei n gleich 2 ist und jedes R unahängig voneinander Chlor darstellt, das an der 3- und 5-Position des Phenylrings substituiert ist.

4. Verfahren nach einem der vorhergehender Ansprüche, wobei das γ-Aluminiumoxid χ-Aluminiumoxid ist, Boehmit oder eine Kombination derselben.

5. Verfahren nach einem der vorhergehenden Ansprüche, wobei das α-Halogenalkylbenzol mit dem γ-Aluminiumoxid in Kontakt gebracht wird in Gegenwart eines Kohlenwasserstoff- oder Halogenkohlenstofflösungsmittels.

6. Verfahren nach einem der vorhergehenden Ansprüche, wobei das γ-Aluminiumoxid durch Erhitzen von Gibbsit bei einer Temperatur von 100 bis 150°C erhalten wird.

7. Verfahren nach Anspruch 5 wobei das γ-Aluminiumoxid durch azeotropes Entfernen des Wassers durch Destillierung des Kohlenwasserstoff- oder Halogenkohlenstofflösungsmittels erhalten wird.

8. Verfahren nach einem der vorhergehenden Ansprüche, das bei Temperaturen zwischen 40 und 160°C durchgeführt wird.

## Revendications

1. Procédé de préparation d'un styrène α-substitué de formule 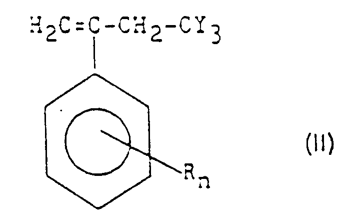 dans laquelle R représente un atome d'halogène, n vaut de 1 à 3, et chaque Y représente indépendamment un atome d'halogène,
qui comporte le fait de faire réagir, en l'absence de tout acide de Lewis supplémentaire, un catalyseur à base de γ-alumine, dont la teneur en humidité vaut entre 0 et 12 % en poids, avec un α-halogénoalkylbenzène de formule 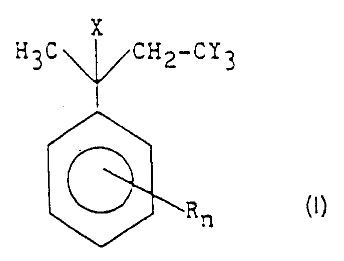 dans laquelle X représente un atome d'halogène et R, n et Y ont les significations indiquées ci-dessus.

2. Procédé conforme à la revendication 1, dans lequel R représente un atome de chlore, X représente un atome de chlore ou de brome, et -Y₃ représente -Cl₃ ou -Br₃.

3. Procédé conforme à la revendication 2, dans lequel n vaut 2, chaque R représente individuellement un atome de chlore, et ces atomes sont placés, en tant que substituants, en les positions 3 et 5 du cycle benzénique.

4. Procédé conforme à l'une des revendications précédentes, dans lequel la γ-alumine est de la χ-alumine, de la boehmite ou une combinaison de celles-ci.

5. Procédé conforme à l'une des revendications précédentes, dans lequel on met l'α-halogénoalkylbenzène en contact avec la γ-alumine en présence d'un solvant de type hydrocarbure ou hydrocarbure halogéné.

6. Procédé conforme à l'une des revendications précédentes, dans lequel on obtient la γ-alumine en chauffant de la gibbsite à une température de 100°C à 150°C.

7. Procédé conforme à la revendication 5, dans lequel on obtient la γ-alumine en éliminant l'eau par voie azéotropique en distillant le solvant de type hydrocarbure ou hydrocarbure halogéné.

8. Procédé conforme à l'une des revendications précédentes, qui est effectué à des températures situées entre 40°C et 160°C.
